# EUROPEAN PATENT APPLICATION

(11) **EP 4 800 419 A1**
(43) Date of publication of application: **02.09.2026**
(21) Application number: 25160928.5
(22) Date of filing: 28.02.2025
(51) Int. Cl.: G01R 33/56, G01R 33/563, G06T 7/11, A61B 5/00, G01R 33/565

(54) **COMPUTER-IMPLEMENTED METHOD TO DETECT AND RE-ACQUIRE CORRUPTED DIFFUSION-WEIGHTED MAGNETIC RESONANCE IMAGING DATA**

(71) Applicant: Siemens Healthineers AG, 91301 Forchheim (DE); King's College London, London WC2R 2LS (GB)
(72) Inventor: MCELROY, Sarah, KT5 9HB London (GB)
(74) Representative: Siemens Healthineers Patent Attorneys

(57) **Abstract**

The invention relates to a computer-implemented method to detect corrupted diffusion-weighted magnetic resonance imaging (DW-MRI) data, comprising: receiving at least one DW-MRI data set (7) describing at least a part of an object and comprising multiple sectional images; determining at least one segmented data set (10) describing for each one of the multiple sectional images of the DW-MRI data set (7) a part assigned to an object part (30) of interest of the object;
- for each sectional image of the segmented data set (10): determining a first corruption verification value (12); if the first corruption verification value (12) fulfills a first corruption condition (13), classifying the sectional image as corrupted;
and/or
- for the segmented data set (10): determining a second corruption verification value (15); if the second corruption verification value (15) fulfills a second corruption condition (16), classifying the segmented data set (10) as corrupted.

## Description

The invention relates to a computer-implemented method and to a method to detect corrupted diffusion-weighted magnetic resonance imaging (DW-MRI) data. Furthermore, the invention relates to a data processing system to perform the computer-implemented method, a magnetic resonance imaging (MRI) system to perform the method and a computer program product to perform the computer-implemented method.

DW-MRI is an imaging technique that uses MRI to measure and display a diffusion movement of water molecules in body tissue. DW-MRI may be used, for example, to examine a brain, because the diffusion behavior in the tissue in the brain may be influenced by a disease of the central nervous system, and/or because directional dependences of the diffusion may allow conclusions about the course of large nerve fiber bundles in the brain. Like classic MRI, DW-MRI is non-invasive and the image contrast is achieved solely by means of magnetic field gradients.

DW-MRI requires an object of interest, such as the brain of a patient, to be essentially motionless during the acquisition of DW-MRI data. In case of motion of the object of interest, the acquired DW-MRI data may comprise at least one motion artefact resulting in DW-MRI data loss. Therefore, DW-MRI data with at least one artefact may be understood as corrupted DW-MRI data.

To detect and/or correct corrupted DW-MRI data, several motion correction techniques are known. Some of the correction techniques are retrospective techniques applied to the DW-MRI data after acquisition. An example for a retrospective motion correction technique is described in the publication "An integrated approach to correction for off-resonance effects and subject movement in diffusion MR imaging" by J. L. Andersson and S. N. Sotiropoulos (Neuroimage, volume 125, pages 1063 to 1078, 2016). Besides, there are prospective motion correction techniques. An example for a prospective motion correction technique is described in the publication "Real-time optical motion correction for diffusion tensor imaging" by M. Aksoy, et al. (Magnetic Resonance in Medicine, volume 66, pages 366 to 378, 2011).

However, known motion correction techniques are often inefficient or inapplicable in specific use cases. This is, for example, the case for fetal brain imaging where specific challenges arise, for example, due to air-tissue interfaces and/or unpredictable motion of the fetus.

It is the object of the invention to improve motion correction for DW-MRI data that is particularly affected by motion artefacts.

The independent claims solve the object.

A first aspect of the invention relates to a computer-implemented method to detect corrupted diffusion-weighted magnetic resonance imaging (DW-MRI) data. A data processing system of a magnetic resonance imaging (MRI) system and/or an external data processing system that receives data from the MRI system may perform the computer-implemented method. Corrupted DW-MRI data in the sense of the invention is DW-MRI data that comprises at least one motion artefact or multiple motion artefacts. A motion artefact is caused by motion of an object or part of an object of which DW-MRI data is acquired during DW-MRI data acquisition.

The computer-implemented method comprises receiving at least one DW-MRI data set. In a preferred example, multiple DW-MRI data sets are received. The at least one DW-MRI data set describes at least a part of an object. The object or the at least part of the object may be a body or a body part of a person, for example. In case of fetal imaging, the at least part of the object may be a fetus or at least a part of a head of the fetus. The computer-implemented method is not limited to fetal imaging. It may be applied to DW-MRI data of any object or part of an object that may be in motion at least temporarily during DW-MRI data acquisition, such as an adult or child brain, a kidney or another organ or body part.

The at least one DW-MRI data set comprises multiple sectional images. The respective sectional image may be understood as a slice of a diffusion-weighted magnetic resonance (DW-MR) image. The section image may be a two-dimensional image that describes the at least part of the object in a specific section plane.

In a preferred example, the at least one DW-MRI data set was acquired under consideration of an acquisition parameter set that is the same for the entire DW-MRI data set. If multiple DW-MRI data sets are received, each one of the multiple DW-MRI data sets was acquired under consideration of an acquisition parameter set that is the same for the entire respective DW-MRI data set. However, different DW-MRI data sets were acquired under consideration of different acquisition parameter sets, for example. In this example, each DW-MRI data set was acquired under consideration of an individual DW-MRI data set. The acquisition parameter set may comprise at least one acquisition parameter, such as a b-value and/or a b-vector. Further acquisition parameters may be possible.

The computer-implemented method comprises determining at least one segmented data set by applying a segmentation algorithm to the at least one DW-MRI data set. If there are multiple DW-MRI data sets, one segmented data set is determined per DW-MRI data set. The segmented data set describes for each one of the multiple sectional images of the at least one DW-MRI data set a part of the at least part of the object that is assigned to an object part of interest of the at least part of the object. In other words, the at least one DW-MRI data set is segmented to determine the part of the at least one DW-MRI data set that describes the object part of interest. The at least one segmented data set allows for each sectional image of the segmented data set to distinguish pixels of the sectional image that describe the object part of interest from other pixels of the sectional image that describes a rest of the at least part of the object and/or at least one surrounding object. The segmented data set comprises multiple sectional images in each of which the object part of interest is identified, for example, by a marking. In case of a fetal imaging, the object part of interest may be the fetal brain. The expressions "object part of interest" and "object part" are used synonymously in the following.

The segmentation algorithm may comprise at least one artificial neural network. The artificial neural network was trained before performing the computer-implemented method to perform a segmentation of sectional images of a DW-MRI data set. Therefore, applying the segmentation algorithm results in an output that describes which pixels of each sectional image correspond to the object part of interest. The segmentation algorithm may be an nnU-NET as described in the publication "nnU-Net: a self-configuring method for deep learning-based biomedical image segmentation" by F. Isensee, et al. (Nature methods, volume 18, pages 203 to 211, 2021). Other segmentation algorithms that are designed to segment an object part of interest in DW-MRI data may be applied alternatively or additionally to nnU-NET.

The computer-implemented method may comprise the following steps for each sectional image of the segmented data set: A first corruption verification value is determined under consideration of a signal intensity determined for the object part of interest in the sectional image. The signal intensity is a value that may be calculated for each pixel of a respective sectional image. Here, it may be calculated for the pixels that describe and are hence assigned to the object part of interest. The signal intensity may be understood as a measured amplitude or strength of the signal an MRI scanner of the MRI system receives from the object part when acquiring the DW-MRI data set for the at least part of the object. Typically, the higher the determined signal intensity the brighter or whiter the pixel appears. The lower or weaker the signal intensity, the darker or blacker the pixel appears. In case of a low signal intensity determined for the object part in the respective sectional image compared to, for example, a signal intensity determined for the object part across the segmented data set, the sectional image may be considered corrupted because it may appear completely or almost completely black and is thus corrupted. This explains why the first corruption verification value that considers signal intensity may be a useful value to detect corrupted sectional images.

After determining the first corruption verification value it may be verified if the first corruption verification value fulfills a predetermined first corruption condition. The first corruption condition may depend, for example, on the object part of interest. The predetermined first corruption condition may be a fixed range, meaning a defined or constant range. In an alternative example, the first corruption condition may be a dynamic range depending on, for example, the at least one acquisition parameter. Alternatively or additionally, the first corruption condition may describe at least one threshold and/or at least one predetermined value. The first corruption condition may be determined by a first corruption condition determining algorithm, that may comprise at least one rule and/or may be based on machine learning techniques. The first corruption condition determining algorithm may determine a value range and/or at least one value based on which it may be determined if the first corruption verification value describes a corrupted sectional image or not.

If the first corruption verification value fulfills the predetermined first corruption condition, the method comprises classifying the sectional image as corrupted. Consequently, a corrupted sectional image is determined or detected. Determining if a respective sectional image of the segmented data set is corrupted or not may be performed for each sectional image individually. If there are multiple DW-MRI data sets and therefore multiple segmented data sets each sectional image of each one of the segmented data sets may be considered individually.

Alternatively or additionally to the steps for detecting the corrupted sectional image, the computer-implemented method may comprise the following steps for the at least one segmented data set. The following steps may be performed for each segmented data set in case of multiple segmented data sets. A second corruption verification value may be determined under consideration of a volume change of a volume of the object part of interest determined for the segmented data set. The volume of the object part of interest may be understood as a three-dimensional size of the object part of interest across all sectional images of the respective segmented data set. The volume of the object part of interest may be determined based on all pixels assigned to the object part of interest in all sectional images of the segmented data set. The volume change may be defined as a change in volume compared to, for example, a reference volume of the object part of interest. The reference volume may be determined based on reference data. The reference data may be or may be comprised by a reference data set. The second corruption verification value allows to detect shifts in the position of the object part of interest in the sectional images of the segmented data set and thus motion of the object part of interest that may cause the segmented data set to contain low or even no meaningful information about the object part of interest. Therefore, the second corruption verification value may be considered to detect corrupted segmented data sets.

After determining the second corruption verification value it may be verified if the second corruption verification value fulfills a predetermined second corruption condition. The first corruption condition and the second corruption condition may differ from one another. The second corruption condition is in a preferred example a dynamic value or range. In this example, the second corruption condition may at least depend, for example, on the object part of interest and/or the at least one acquisition parameter. In an alternative example, the second corruption condition may be a fixed range, meaning a defined or constant range. If the second corruption verification value fulfills the predetermined second corruption condition, the method comprises classifying the segmented data set as corrupted. Consequently, a corrupted segmented data set is determined or detected. Alternatively or additionally, the second corruption condition may describe at least one threshold and/or at least one predetermined value. The second corruption condition may be determined by a second corruption condition determining algorithm, that may comprise at least one rule and/or may be based on machine learning techniques. The second corruption condition determining algorithm may determine a value range and/or at least one value based on which it may be determined if the second corruption verification value describes a corrupted sectional image or not. The second corruption condition determining algorithm may differ at least partially from the first corruption condition determining algorithm.

As a result of the computer-implemented method, at least one corrupted sectional image of at least one segmented data set and/or at least one corrupted segmented data set are determined or detected. This is achieved by considering the first corruption verification value to detect motion-induced signal loss and/or the second corruption verification value to detect through-sectional image motion within one segmented data set. The DW-MRI data set based on which the at least one corrupted sectional image and/or the at least one corrupted segmented data set are determined may be referred to as corrupted DW-MRI data.

The computer-implemented method determines precisely, for example, while receiving consecutively DW-MRI data sets, if the currently considered DW-MRI data set comprises at least one corrupted sectional image and/or segmented data set or not. This classification may be performed essentially in real time after receiving a respective DW-MRI data set so that live-detection of corrupted DW-MRI data is possible. The detection of corrupted DW-MRI data according to the computer-implemented method is completely automated so than no manual contribution is necessary. The described computer-implemented method is particularly useful for strongly and/or unpredictably moving objects or object parts of interest such as fetal brains.

An embodiment comprises that, if at least one of the sectional images and/or the at least one segmented data set is classified as corrupted, a reacquisition information is determined. The reacquisition information may be sent to the MRI scanner and may be understood as a control command that may be executed by the MRI scanner, for example. The reacquisition information describes a request for reacquisition of DW-MRI data, meaning of at least one DW-MRI data set or a part of the at least one DW-MRI data set, based on which the at least one corrupted sectional image is replaceable by at least one re-determined sectional image. Alternatively or additionally, the reacquisition information describes a request for reacquisition of DW-MRI data, meaning of at least one DW-MRI data set or a part of the at least one DW-MRI data set, based on which the segmented data set that comprises the at least one corrupted sectional image is replaceable by a re-determined segmented data set. Alternatively or additionally, the reacquisition information describes a request for reacquisition of DW-MRI data, meaning of at least one DW-MRI data set or a part of the at least one DW-MRI data set, based on which the at least one corrupted segmented data set is replaceable by at least one re-determined segmented data set. The reacquisition information thus asks for data reacquisition to acquire uncorrupted DW-MRI data based on which the corrupted sectional image and/or the corrupted segmented data set may be compensated. It is hereby assumed that the re-determined segmented data set and/or the re-determined sectional image may comprise no motion artifacts and is thus uncorrupted. However, the above-described computer-implemented method may be re-performed for the reacquired DW-MRI data to verify that the corrupted sectional image and/or segmented data set are not replaced by another corrupted sectional image and/or segmented data set but by an uncorrupted sectional image and/or segmented data set. Consequently, the computer-implemented method may not only detect corrupted DW-MRI data but also organize reacquisition of the corrupted DW-MRI data.

Another embodiment comprises that the reacquisition information describes which DW-MRI data, meaning which DW-MRI data set or part of a DW-MRI data set, has to be reacquired by at least one acquisition parameter for the reacquisition. In other words, the reacquisition information specifies how to reacquire the DW-MRI data that is then processed to replace the corrupted sectional image and/or segmented data set. The at least one acquisition parameter may be comprised by the acquisition parameter set. Using the acquisition parameter to define the reacquisition is particularly useful to avoid reacquiring not needed DW-MRI data, such as not needed DW-MRI data sets. The reacquisition information thus comprises precise commands for the MRI scanner that may be used by the MRI scanner to perform fast and precise reacquisition of DW-MRI data to replace only the at least one corrupted sectional image and/or segmented data set.

In a preferred embodiment the at least one acquisition parameter is a b-value and/or a b-vector. The b-value may be a factor that reflects timing and/or strength of a diffusion gradient used to generate the DW-MRI data set. Typically, a higher b-value relates to stronger diffusion effects compared to a lower b-value. The b-vector may describe a direction with which the DW-MRI data is acquired. The b-value and the b-vector are reasonable information that specify the acquisition of DW-MRI data.

Alternatively or additionally, the at least one acquisition parameter may be a sectional image characterization such as, for example, a number or other kind of identification to identify at least one sectional image. The sectional image characterization thus identifies at least one sectional image that is to be reacquired to replace the at least one corrupted sectional image. This is advantageous if reacquisition of individual sectional images and thus of only a part of a DW-MRI data set is intended.

An embodiment comprises that the first corruption verification value is defined as a ratio of a mean signal intensity of the object part in the sectional image and a mean signal intensity of the object part across all sectional images of the segmented data set. The first corruption verification value is thus calculated by dividing the mean signal intensity of the object part in the sectional image by the mean signal intensity of the object part across all sectional images of the segmented data set. This means that the first corruption verification value is determined under consideration of the mean signal intensity of the object part in the sectional image and the mean signal intensity of the object part across all sectional images of the segmented data set. This means that, for example, the signal intensity of each pixel assigned to the object part of interest in the respective sectional image may be summed and divided by a total number of summed pixels of the respective sectional image to determine the mean signal intensity for the object part in the respective sectional image. In case the sectional image describes the fetal brain, the mean signal intensity of only the pixels that describe the fetal brain in the sectional image are considered when determining the mean signal intensity. The mean signal intensity of the object part across all sectional images may be determined under consideration of the signal intensities determined for all sectional images of the segmented data set that comprise the sectional image for which the first corruption verification value is determined. The mean signal intensity of the object part across all sectional images of the segmented data set may be understood as the total mean signal intensity of the object part of interest in the segmented data set. The described definition of the first corruption verification value is particularly easy and fast to determine so that corrupted sectional images in segmented data sets are easily and fast determined.

In a preferred example, the first corruption condition is a first range that is between 0 and 0.35. Different, higher or lower first ranges may be possible. The first corruption condition may be defined by only one upper or lower threshold, for example. In a preferred example, the first corruption condition is given by the upper threshold so that any value below this upper threshold is considered to fulfill the first corruption condition. In this example, the first corruption condition may be defined by the upper threshold of 0.35. If the first corruption verification value is below the upper threshold, the sectional image is classified corrupted. The first corruption condition as the first range between 0 and 0.35 is particularly useful for imaging of fetal brains.

The upper threshold of 0.35 means that the mean intensity of the sectional image is 35 percent or less than the mean volumetric intensity, meaning the mean signal intensity of the object part across all sectional images of the segmented data sets, to classify the sectional image corrupted. The first corruption condition between 0 and 0.35 was selected as a balance between motion-induced signal loss and naturally lowers signal intensities in certain parts of the object part, such as certain brain structures like cortical boundaries and/or ventricles in the brain.

According to another embodiment, the second corruption verification value is defined as a ratio of a volume of the object part across all sectional images of the segmented data set and a volume of the object part across all sectional images of a reference data set. The reference data set may be a segmented data set based on which the reference volume of the object part is determined. It is hereby assumed that the reference data set is captured without motion of the object part so that the reference data set may be understood as a segmented data set that shows no or essentially no motion artefacts. The second corruption verification value is thus calculated, for example, by dividing the volume of the object part across all sectional images of the segmented data set by the volume of the object part across all sectional images of the reference data set. The described definition of the second corruption verification value is particularly easy and fast to determine so that corrupted segmented data sets are easily and fast determined.

A further embodiment comprises that the reference data set is a segmented data set that is determined based on a first acquired DW-MRI data set and/or based on a DW-MRI data set that was acquired at a b-value of 0. The first acquired DW-MRI data set may be the first received DW-MRI data set for the at least part of the object. It may alternatively be referred to as a starting DW-MRI data set. It is hereby assumed that typically the first acquired DW-MRI data set is not corrupted by motion at least with a high probability compared to afterwards acquired DW-MRI data sets. The DW-MRI data set that was acquired at a b-value of 0 may be understood as an MRI-data set acquired without applying diffusion-weighting so that the b-value is kept at 0. Alternatively to the b-value of 0, another b-value may be set for the reference data set.

It is assumed that the reference data set allows to determine a volume of the object part that is equal to or at least essentially equal to the actual volume of the object part. Therefore, the volume of the object part across the currently considered data set, meaning the volume across all sectional images of the segmented data set, is compared to the expected or assumed actual volume of the object part, meaning the volume of the object part across the reference data set, to decide if motion occurred that resulted in a volume change because of which the segmented data set is corrupted. The reference data set used is particularly suitable as such.

Besides, an embodiment comprises that the predetermined second corruption condition depends at least on a b-value at which the DW-MRI data set was acquired based on which the considered segmented data set was determined. The b-value is hereby the b-value assigned to the DW-MRI data set based on which the segmented data set was determined for which the second corruption verification value is determined and compared to the second corruption condition. Alternatively or additionally, the predetermined second corruption condition may depend on an apparent diffusion coefficient (ADC) of a fluid in and/or around the object part of interest. In case of the fetal brain as object part of interest, the ADC may be adapted to the cerebrospinal fluid (CSF) that surrounds the fetal brain and exhibits high diffusivity compared to other parts of the fetal brain. An example for the ADC for the fetal brain may be 0.002 square millimeter per second. At higher b-values, the signal from the CSF may attenuate rapidly which may decrease a segmentation accuracy of the segmentation algorithm. Alternatively or additionally, the second corruption condition may depend on at least one empirically determined parameter. The at least one empirically determined parameter may be referred to as a scaling factor α. The empirically determined parameter, meaning the scaling factor, may be optimized to maintain sensitivity to biologically significant changes and robustness to noise. In a preferred example for the fetal brain, the empirically determined parameter α is 0.3.

The second corruption condition may be defined by only one upper or lower threshold, for example. In a preferred example, the second corruption condition is given by the upper threshold so that any value below this upper threshold is considered to fulfil the second corruption condition. In the example with the fetal brain as object part of interest, the upper threshold of the second corruption condition may be calculated by the following formula:$T = α * \left(1-e-b*ADC\right) + 0.02 ,$ with T for the upper threshold, b for the b-value, the above-described empirically determined parameter α and the above-described ADC.

The second corruption condition may be a second range that may be between 0 and T, wherein T is a function of the b-value assigned to the DW-MRI data set based on which the segmented data set is determined for which the second corruption verification value is determined and verified. The formula may be different for another object part that is no fetal brain. In summary, a reasonable second corruption condition may be determined for the respective segmented data set to detect the corrupted segmented data set reliably.

Another embodiment comprises that multiple DW-MRI data sets are received. For each one of the multiple received DW-MRI data sets a center point of the object part is determined by applying a center point determination algorithm to the segmented data set that is determined based on the DW-MRI data set. The center point determination algorithm may comprise at least one rule and/or at least one trained artificial neural network that is applied to the multiple DW-MRI data sets to calculate the center point of the object part for each DW-MRI data set of the multiple DW-MRI data sets. The center point determination algorithm may rely on known techniques for determining a center point of an object or object part in DW-MRI data.

The embodiment comprises that a respective spatial shift of the center points between consecutive segmented data sets is determined. If the respective spatial shift is below a predetermined shift threshold, the first corruption verification value and/or the second corruption verification value are determined. In a preferred example, the first corruption verification value and/or the second corruption verification value are only determined for segmented data sets or sectional images of segmented data sets, if the spatial shift between these segmented data sets and consecutive segmented data sets is below the predetermined threshold. The spatial shift may be understood as an average Euclidean distance between the center points of the object parts in two segmented data sets that were acquired directly after another.

If the spatial shift is higher than or equal to the predetermined shift threshold motion between the consecutively acquired DW-MRI data sets is considered to be too strong for the computer-implemented method so that the computer-implemented method may not be suitable or applicable to the DW-MRI data sets. This embodiment may thus help to avoid performing the computer-implemented method in situations that are outside a scope of application of the computer-implemented method.

Another aspect of the invention relates to a method to detect corrupted DW-MRI data. The method comprises acquiring at least one DW-MRI data set by the MRI scanner of the MRI system. The at least one DW-MRI data set describes at least a part of an object and comprises multiple sectional images. In a preferred example, each DW-MRI data set is acquired under consideration of an acquisition parameter set that is the same for the entire DW-MRI data set and comprises at least one acquisition parameter, such as the b-value and/or the b-vector. Afterwards, the method comprises performing the above-described computer-implemented method.

A preferred embodiment of the method comprises reacquiring DW-MRI data to replace the detected corrupted DW-MRI data. The method comprises receiving the reacquisition information described above by the DW-MRI scanner and reacquiring the DW-MRI data according to the reacquisition information by the MRI scanner. Consequently, uncorrupted DW-MRI data may be available for further processing. The computer-implemented method may be performed for the reacquired DW-MRI data as well to ensure that the reacquired DW-MRI data is not corrupted.

A further embodiment of the method comprises that before acquiring the at least one DW-MRI data set a planning algorithm is performed. The planning algorithm is performed to determine an axial scanning plane for the acquisition of the at least one DW-MRI data set, so that the acquired at least one DW-MRI data set describes a predetermined object of interest as the at least part of the object. Acquiring of the at least one DW-MRI data set may be performed under consideration of the determined axial scanning plane. The planning algorithm may be based on known planning techniques to automatically plan a DW-MRI data acquisition. An example for such a planning algorithm for fetal brain imaging is described in the publication "Real-time fetal brain tracking for functional fetal MRI" by S. Neves Silva, et al. (Magnetic resonance in medicine, volume 90, pages 2306 to 2320, 2023). This planning algorithm may comprise that acquired sectional images are processed using an artificial neural network to localize the object part of interest which may be the fetal brain. This is achieved by identifying specific landmarks in and/or around the object part of interest, such as the fetal brain. Based on the identified landmarks, the planning algorithm identifies the object part of interest and determines the axial scanning plane suitable for acquiring DW-MRI data of the object part of interest. The landmarks may be understood as characteristics of and/or around the object part of interest used to detect the object part of interest in DW-MRI data.

Another aspect of the invention relates to a data processing system configured to perform a computer-implemented method as described above. The data processing system may be comprised by the MRI system.

Unless stated otherwise, all steps of the computer-implemented method may be performed by the data processing system, which comprises at least one data processing device. In particular, the at least one data processing device is configured or adapted to perform the steps of the computer-implemented method. For this purpose, the at least one data processing device may for example store a computer program comprising instructions which, when executed by the at least one data processing device, cause the at least one data processing device to execute the computer-implemented method. The expressions "data processing system" and "at least one data processing device" may be used interchangeably, here and in the following. This holds also for respective expressions derived therefrom.

In case the at least one data processing device comprises two or more data processing devices, certain steps carried out by the at least one data processing device may also be understood such that different data processing devices carry out different steps or different parts of a step. In particular, it is not required that each data processing device carries out the steps completely. In other words, carrying out the steps may be distributed amongst the two or more data processing devices.

From each implementation of the computer-implemented method, a respective implementation of the method, which is not purely computer-implemented, is obtained by including respective steps of acquiring and/or reacquiring DW-MRI data.

Another aspect of the invention relates to an MRI system that comprises an MRI scanner and a data processing system as described above. The MRI system is configured to perform the above-described method. The MRI system performs the above-described method.

According to a further aspect of the invention, a computer program comprising instructions is provided. When the instructions are executed by a data processing system, the instructions cause the data processing system to carry out a computer-implemented method according to the invention.

The instructions may be provided as program code, for example. The program code can for example be provided as binary code or assembler and/or as source code of a programming language, for example C, and/or as program script, for example Python.

According to a further aspect of the invention, a computer-readable storage medium, in particular a tangible and/or non-transient computer readable storage medium, storing a computer program according to the invention is provided.

The computer program and the computer-readable storage medium are respective computer program products comprising the instructions.

Receiving data or information within the meaning of the invention may, for example, involve receiving or obtaining the data or information, in particular by the data processing system from a transmitting entity, or reading the data from a data memory, or receiving a data stream containing the data or the information, or extracting the data from the data stream. In particular, wired or wireless data transmission may be used for this purpose. In particular, the data transmission may take place between a hardware and/or software interface of the sending entity and a hardware and/or software interface of the data processing system.

Independent of the grammatical term usage, individuals with male, female or other gender identities are included within the term.

Further features of the invention are apparent from the claims, the figures and the figure description. The features and combinations of features mentioned above in the description as well as the features and combinations of features mentioned below in the description of figures and/or shown in the figures may be comprised by the invention not only in the respective combination stated, but also in other combinations. In particular, embodiments and combinations of features, which do not have all the features of an originally formulated claim, may also be comprised by the invention. Moreover, embodiments and combinations of features which go beyond or deviate from the combinations of features set forth in the recitations of the claims may be comprised by the invention.

In the following, the invention will be explained in detail with reference to specific exemplary implementations and respective schematic drawings. In the drawings, identical or functionally identical elements may be denoted by the same reference signs. The description of identical or functionally identical elements is not necessarily repeated with respect to different figures.

The figures show in:
FIG 1 a schematic representation of a diffusion-weighted magnetic resonance imaging system;
FIG 2 a schematic representation of a method and a computer-implemented method to detect corrupted diffusion-weighted magnetic resonance imaging data; and
FIG 3 an example of a corrupted and an uncorrupted diffusion-weighted magnetic resonance imaging data set.

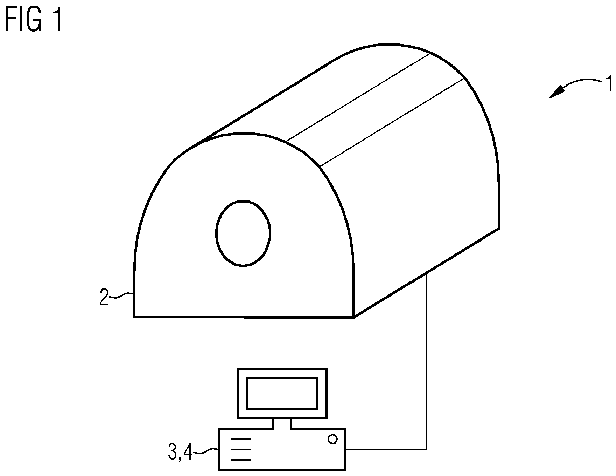

FIG 1 shows schematically an exemplary implementation of a magnetic resonance imaging (MRI) system 1 according to the invention. The MRI system 1 comprises an MRI scanner 2 and a data processing system 3 for controlling the MRI scanner 2. Furthermore, the MRI system 1 comprises a computing unit 4 coupled to the data processing system 3 and/or to the MRI scanner 2. Alternatively, the computing unit 4 may comprise the data processing system 3 or vice versa. The MRI system 1 may also comprise a storage unit (not shown) storing a computer program according to the invention.

The MRI system 1 may be used to carry out a method for diffusion-weighted MRI (DW-MRI) according to the invention. In particular, the data processing system 3 may execute the computer program according to the invention. Therein, the data processing system 3 may carry out an exemplary implementation of a computer-implemented method according to the invention.

In general, DW-MRI may be considered to exploit the attenuation of a respective MRI-signal, based on the diffusion of water molecules in a region, meaning an object, to be imaged. The more diffusion occurs, the further a water molecule may move within a given period of time, which results in a reduction of the MRI-signal. For example, cerebrospinal fluid (CSF) comprises water, which may diffuse rather easily so that the respective image regions may appear dark or black. On the other hand, water within tissues may not move as easily. Thus, a respective contrast may be achieved.

For DW-MRI, one or more b0-images may be obtained. These are MRI-data sets acquired without applying diffusion-weighting, meaning with b = 0, wherein b = γ2*G2*δ2*(Δ - δ/3). Therein, γ denotes the gyromagnetic ratio, G denotes the amplitude of diffusion gradient lobes, δ denotes their respective duration and Δ denotes a duration between them.

Furthermore, one or more images with b > 0, for example with b in the interval [100 s/mm2, 10000 s/mm2] or in the interval [200 s/mm2, 5000 s/mm2], are obtained. In particular, these may be obtained for different spatial directions of the diffusion gradients. Each spatial direction in combination with the used b-value defines a point in a three-dimensional space denoted as Q-space.

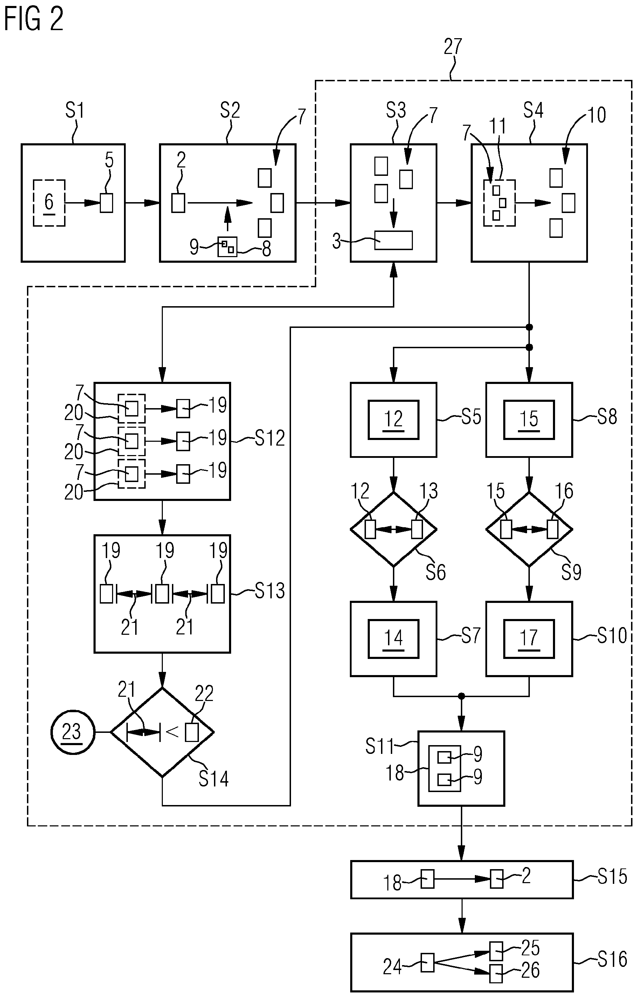

FIG 2 shows steps of a method and a computer-implemented method to detect corrupted DW-MRI data. The steps of the computer-implemented method are sketched within a box 27. The computer-implemented method may be performed by the data processing system 3 of the MRI system 1. An acquisition of at least one DW-MRI data set 7 may be performed by the MRI scanner 2.

In a step S1, a planning algorithm 6 may be performed to determine an axial scanning plane 5 for the acquisition of the at least one DW-MRI data set 7 so that the acquired at least one DW-MRI data set 7 describes at least a part of an object that is an object of interest. Acquiring of at least one DW-MRI data set 7 in a step S2 may be performed under consideration of the determined axial scanning plane 5. Acquiring the at least one DW-MRI data set 7 may be performed by the MRI scanner 2. The at least one acquired DW-MRI data set 7 describes at least a part of the object and comprises multiple sectional images. In a preferred example, the DW-MRI data set 7 is acquired under consideration of an acquisition parameter set 8 that is the same for the entire DW-MRI data set 7. The acquisition parameter set 8 may comprise at least one acquisition parameter 9. The acquisition parameter 9 may be, for example, a b-value and/or a b-vector. In a step S3, the computer-implemented method may start with receiving the at least one DW-MRI data set 7.

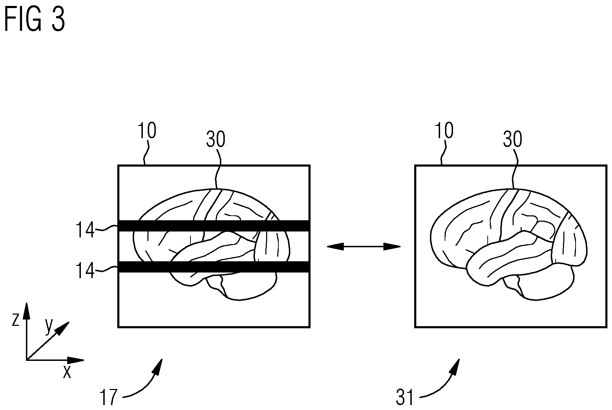

In a step S4, the computer-implemented method may comprise determining at least one segmented data set 10 by applying a segmentation algorithm 11 to the at least one DW-MRI data set 7. The segmented data set 10 describes for each one of the multiple sectional images of the DW-MRI data set 7 a part that is assigned to an object part 30 (see reference signs 30 in FIG 3) of interest of the at least part of the object. The segmentation algorithm 11 may comprise at least one artificial neural network, in particular an nnU-NET or another segmentation approach. In an example, the object may be a fetus in a placenta of a female patient. The object part 30 may then, for example, be a fetal brain of the fetus. Other examples for objects and/or object parts 30 are possible.

In a step S5, a first corruption verification value 12 may be determined for each sectional image of the at least one segmented data set 10. If there are multiple DW-MRI data sets 7, multiple segmented data sets 10 may be determine in the step S4. The first corruption verification value 12 may be determined under consideration of a signal intensity determined for the object part 30 in the sectional image. For example, the first corruption verification value 12 may be defined as a ratio of a mean signal intensity of the object part 30 in the sectional image and a mean signal intensity of the object part 30 across all sectional images of the segmented data set 10.

In a step S6, it may be verified if the first corruption verification value 12 fulfills a predetermined first corruption condition 13. In case of the fetal brain, the first corruption condition 13 may be a first range that may be between 0 and 0.35. In another formulation, the first corruption condition 13 may be understood as an upper threshold that is, for example, 0.35 so that if the first corruption verification value 12 is below 0.35, the first corruption verification value 12 is considered to fulfill the predetermined first corruption condition 13. If this is the case, the sectional image may be classified as corrupted in a step S7. This means that at least one corrupted sectional image 14 is determined. The steps S5 to S7 may be performed for each sectional image of each segmented data sets 10. As a result, there may be multiple corrupted sectional images 14 detected.

Alternatively or additionally to the steps S5 to S7, the following steps S8 to S10 may be performed for each segmented data set 10. In step S8, a second corruption verification value 15 may be determined. The second corruption verification value 15 may be determined under consideration of a volume change of a volume of the object part 30 determined for the segmented data set 10. For example, the second corruption verification value 15 may be defined as a ratio of a volume of the object part 30 across all sectional images of the segmented data set 10 and a volume of the object part 30 across all sectional images of a reference data set. The reference data set may be a segmented data set 10 that is determined based on a first acquired DW-MRI data set 7 and/or a DW-MRI data set 7 that was acquired at a b-value of 0, meaning at b = 0.

In a step S9, it may be verified if the second corruption verification value 15 fulfills a predetermined second corruption condition 16. The second corruption condition 16 may be given as an upper threshold that in particular may defines an upper end of a second range. In this case, the second corruption condition 16 may be between 0 and the upper threshold. In a step S10 the segmented data set 10 is classified as corrupted, if the second corruption verification value 15 fulfills the predetermined second corruption condition 16. This means that in the step S10 at least one corrupted segmented data set 17 may be determined. If there are multiple DW-MRI data sets 7 and thus multiple segmented data sets 10 it is possible to determine multiple corrupted segmented data sets 17.

In a preferred example, the predetermined second corruption condition 16, in particular the upper threshold of the second corruption condition 16, depends at least on the b-value, at which the DW-MRI data set 7 was acquired based on which the segmented data set 10 was determined. Alternatively or additionally, the predetermined second corruption condition 16 may depend on an apparent diffusion coefficient (ADC) of fluid in and/or around the object part 30 and/or at least one empirically determined parameter. This means that the second corruption condition 16 may be a dynamic value or range that depends at least on the b-value.

After the step S7 and/or the step S10, a step S11 may be performed if at least one of the sectional images and/or at least one segmented data set 10 is classified as corrupted. In the step S11, a reacquisition information 18 may be determined if at least one of the sectional images and/or the at least one segmented data set 10 is classified as corrupted. The reacquisition information 18 may describe a request for the reacquisition of DW-MRI data based on which the at least one corrupted sectional image 14 is replaceable by at least one re-determined sectional image. Alternatively or additionally, the reacquisition information 18 may describe a request for the reacquisition of DW-MRI data based on which the segmented data set 10 that comprises the at least one corrupted sectional image 14 is replaceable by a re-determined segmented data set 10. Alternatively or additionally, the reacquisition information 18 may describe a request for the reacquisition of DW-MRI data based on which the at least one corrupted segmented data set 17 is replaceable by at least one re-determined segmented data set 10.

The reacquisition information 18 may describe which DW-MRI data has to be reacquired by giving at least one acquisition parameter 9 for the reacquisition. The acquisition parameter 9 may be the b-value and/or the b-vector. In case of reacquiring the corrupted sectional image 14, the acquisition parameter 9 may describe a sectional image characterization such as a number or another information to identify which sectional image of the DW-MRI data set 7 has to be reacquired.

After the step S3 further steps S12 to S14 may be performed to decide whether the first corruption verification value 12 and/or the second corruption verification 15 should be determined or not. The step S12 is based on the assumption that multiple DW-MRI data sets 7 are received. For each one of the multiple DW-MRI data sets 7, a center point 19 of the object part 30 is determined by applying a center point determination algorithm 20 to the segmented data set 10 that was determined based on the DW-MRI data set 7. In a step S13, a respective spatial shift 21 of the center point 19 between consecutive segmented data sets 10 is determined. The spatial shift 21 of the center point 19 between consecutive segmented data sets 10 is calculated or measured. If the respective spatial shift 21 is below a predetermined shift threshold 22 in step S14, the first corruption verification value 12 and/or the second corruption verification value 15 are determined. If, however, the respective spatial shift 21 reaches the shift threshold 22 or is higher than the predetermined shift threshold 22, an end 23 of the computer-implemented method may be reached. In this case, the first corruption verification value 12 and/or the second corruption verification value 15 may not be determined, meaning that at least the step S5 and/or the step S8 and following steps may not be performed.

After the step S11 a step S15 may be performed. The step S15 may comprise receiving the reacquisition information 18 by the DW-MRI scanner 2. The DW-MRI scanner 2 may then in a step S16 reacquire the DW-MRI data 24 according to the reacquisition information 18 so that the reacquired DW-MRI data 24 may comprise reacquired sectional images 25 and/or reacquired DW-MRI data sets 26. Afterwards, the steps S3 and the following steps may be performed again to verify if the reacquired DW-MRI data 24 is uncorrupted or not.

FIG 3 shows two examples of segmented data sets 10. The individual sectional images are arranged on top of each other to describe the object part 30. The object part 30 is here the fetal brain. Other object parts 30 are possible. The individual sectional images are two-dimensional images in an x-y-plane. Multiple sectional images are here superimposed to indicate the volume of the object part 30 in z- and x-direction.

On the left side, an example for a corrupted segmented data set 17 is sketched that comprises multiple corrupted sectional images 14. The corrupted sectional images 14 are blackened because of motion of the object part 30. On the right side, an uncorrupted segmented data set 31 is shown, that comprises no corrupted sectional images 14 and no volume change that does not fulfill the second corruption condition 16 so that the uncorrupted segmented data set 31 is well suitable for further analysis.

In summary, the invention describes the high-efficiency real-time motion detection and reacquisition for DW-MRI data. The computer-implemented method aims to provide an efficient and robust solution for DW-MRI by combining:
- automated planning of the diffusion acquisition (step S1);
- automated segmentation of the object part 30 of interest for each b-value and/or direction (step S4);
- metric-based detection of corrupted sectional images 14 and/or segmented data sets 17 (steps S5 to S10); and/or
- prioritized reacquisition of corrupted sectional images 14 and/or segmented data sets 17 (steps S11, S15 and S16).

Identification of the corrupted sectional image 14 and/or corrupted segmented data set 17 may occurs in real-time so no additional delays may be introduced by processing. Applying the planning algorithm 6 may be based on a fast multi-echo gradient echo sequence covering the entire uterus in case of the fetal brain as object part 30 of interest. The hereby acquired images may be processed using an artificial neural network to localize the fetal brain and identify specific landmarks. These landmarks may then be used to calculate the axial scanning plane 5 for the DW-MRI data acquisition.

Detection of motion corruption first requires automatic segmentation in real-time of the object part 30 of interest. For that, an artificial neural network based on the nnU-Net framework may be used. To enhance robustness and to assure broad applicability, the artificial neural network may be trained and validated on a data set of almost 3000 fetal scans incorporating variations across three magnetic field strengths (0.55 Tesla, 1.5 Tesla and 5 Tesla), two MRI systems 1, and five different contrasts (single-shot fast spin echo, balanced steady state free precession, DW-MRI, T1 and T2 maps). The segmented fetal brain is then used to calculate the following matrix for detection of motion and corruption which are here the first corruption verification value 12 and/or the second corruption verification value 15. A threshold value is used to identify a corrupted sectional image 14. This threshold is selected to balance between identifying motion-induced signal loss and allowing for lower signal intensities in certain brain structures such as the cortical boundaries or ventricles, which exhibit lower signal values under normal conditions.

Besides, for the second corruption verification value 15, a dynamic threshold may be used to mitigate for signal attenuation at high b-values. The threshold is modelled as a scaled function of the b-value and the apparent diffusion coefficient (ADC). This approach allows for the detection of meaningful diffusion-related changes minimizing false positives, especially at high b-values. Corrupted sectional images 14 and/or corrupted segmented data set 17 are identified as those where either one of these two metrics (first corruption verification values 12, second corruption verification value 15) exceeds the respective threshold value, meaning does not fulfill the predetermined first corruption condition 13 or second corruption condition 16, respectively.

The reacquisition comprises that DW-MRI data sets 7 at b-values and b-vectors of the identified corrupted sectional images 14 and/or corrupted segmented data sets 17 are reacquired. The process can be repeated as needed, facilitating iterative refinement of data quality while considering acquisition time constraints. An additional automatic planning step may be used before the reacquisition step to improve consistency of the reacquired data with the initial data set. Inter-volume motion is thus corrected using rigid volume registration.

In other words, the invention relates to a computer-implemented method to detect corrupted DW-MRI data, comprising: receiving at least one DW-MRI data set 7 describing at least a part of an object and comprising multiple sectional images; determining at least one segmented data set 10 describing for each one of the multiple sectional images of the DW-MRI data set 7 a part assigned to an object part 30 of interest of the object;
- for each sectional image of the segmented data set 10: determining a first corruption verification value 12; if the first corruption verification value 12 fulfills a first corruption condition 13, classifying the sectional image as corrupted;
   and/or
- for the segmented data set 10: determining a second corruption verification value 15; if the second corruption verification value 15 fulfills a second corruption condition 16, classifying the segmented data set 10 as corrupted.

## Claims

1. Computer-implemented method to detect corrupted diffusion-weighted magnetic resonance imaging (DW-MRI) data, comprising:
- receiving at least one DW-MRI data set (7) that describes at least a part of an object and comprises multiple sectional images;
- determining at least one segmented data set (10) by applying a segmentation algorithm (11) to the at least one DW-MRI data set (7), wherein the segmented data set (10) describes for each one of the multiple sectional images of the DW-MRI data set (7) a part that is assigned to an object part (30) of interest of the at least part of the object;
- for each sectional image of the segmented data set (10),
- determining a first corruption verification value (12) under consideration of a signal intensity determined for the object part (30) in the sectional image;
- verifying if the first corruption verification value (12) fulfills a predetermined first corruption condition (13);
- if the first corruption verification value (12) fulfills the predetermined first corruption condition (13), classifying the sectional image as corrupted;
and/or
- for the at least one segmented data set (10),
- determining a second corruption verification value (15) under consideration of a volume change of a volume of the object part (3) determined for the segmented data set (10);
- verifying if the second corruption verification value (15) fulfills a predetermined second corruption condition (16);
- if the second corruption verification value (15) fulfills the predetermined second corruption condition (16), classifying the segmented data set (10) as corrupted.

2. Computer-implemented method according to claim 1, wherein, if at least one of the sectional images and/or the at least one segmented data set (10) is classified as corrupted, a reacquisition information (18) is determined, wherein the reacquisition information (18) describes a request for reacquisition of DW-MRI data based on which:
- the at least one corrupted sectional image (14) is replaceable by at least one re-determined sectional image; and/or
- the segmented data set (10) that comprises the at least one corrupted sectional image (14) is replaceable by a re-determined segmented data set (10); and/or
- the at least one corrupted segmented data set (17) is replaceable by at least one re-determined segmented data set (10).

3. Computer-implemented method according to claim 2, wherein the reacquisition information (18) describes which DW-MRI data has to be reacquired by at least one acquisition parameter (9) for the reacquisition.

4. Computer-implemented method according to claim 3, wherein the at least one acquisition parameter (9) is a b-value and/or a b-vector and/or a sectional image characterization.

5. Computer-implemented method according to any one of the preceding claims and the first alternative of claim 1, wherein the first corruption verification value (12) is defined as a ratio of a mean signal intensity of the object part (30) in the sectional image and a mean signal intensity of the object part (30) across all sectional images of the segmented data set (10).

6. Computer-implemented method according to any one of the preceding claims and the second alternative of claim 1, wherein the second corruption verification value (15) is defined as a ratio of a volume of the object part (30) across all sectional images of the segmented data set (10) and a volume of the object part (30) across all sectional images of a reference data set.

7. Computer-implemented method according to claim 6, wherein the reference data set is a segmented data set (10) that is determined based on a first acquired DW-MRI data set (7) and/or on a DW-MRI data set (7) that was acquired at a b-value of 0.

8. Computer-implemented method according to any one of the preceding claims and the second alternative of claim 1, wherein the predetermined second corruption condition (16) depends at least on:
- a b-value at which the DW-MRI data set (7) was acquired; and/or
- an apparent diffusion coefficient of a fluid in and/or around the object part (30); and/or
- at least one empirically determined parameter.

9. Computer-implemented method according to any one of the preceding claims, wherein multiple DW-MRI data sets (7) are received, for each one of the multiple DW-MRI data sets (7) a center point (19) of the object part (30) is determined by applying a center point determination algorithm (20) to the segmented data set (10) that is determined based on the DW-MRI data set (7), and a respective spatial shift (21) of the center point (19) between consecutive segmented data sets (10) is determined, wherein if the respective spatial shift (21) is below a predetermined shift threshold (22), the first corruption verification value (12) and/or the second corruption verification value (15) are determined.

10. Method to detect corrupted DW-MRI data, comprising:
- acquiring at least one DW-MRI data set (7) by an MRI scanner (2) of an MRI system (1), wherein the respective at least one DW-MRI data set (7) describes at least a part of an object and comprises multiple sectional images; and
- performing a computer-implemented method according to any one of the preceding claims.

11. Method according to claim 10 in its back-references to claim 2, wherein the method comprises the following steps performed by the MRI scanner (2):
- receiving the reacquisition information (18); and
- reacquiring the DW-MRI data according to the received reacquisition information (18).

12. Method according to claim 10 or 11, wherein before acquiring the at least one DW-MRI data set (7) a planning algorithm (6) is performed to determine an axial scanning plane (5) for the acquisition of the at least one DW-MRI data set (7), so that the acquired at least one DW-MRI data set (7) describes a predetermined object of interest as the at least part of the object, and the acquiring of the at least one DW-MRI data set (7) is performed under consideration of the determined axial scanning plane (5).

13. Data processing system (3) configured to perform a computer-implemented method according to any one of claims 1 to 11.

14. MRI system (1), comprising an MRI scanner (2) and a data processing system (3) according to claim 13, wherein the MRI system (1) is configured to perform a method according to any one of claims 10 to 12.

15. Computer program product comprising instructions, which, when executed by a data processing system (3), cause the data processing system (3) to carry out a computer-implemented method according to any one of claims 1 to 9.
